# EUROPEAN PATENT APPLICATION

(11) **EP 2 578 701 A1**
(43) Date of publication of application: **10.04.2013**
(21) Application number: 12187042.2
(22) Date of filing: 02.10.2012
(51) Int. Cl.: C12Q 1/68

(54) **Probe, polymorphism detection method, method of evaluating drug efficacy or tolerance, and reagent kit**

(30) Priority: 03.10.2011 JP 2011219336; 30.08.2012 JP 2012190186
(71) Applicant: ARKRAY, Inc., Kyoto-shi, Kyoto 601-8045 (JP)
(72) Inventor: Kurose, Kaoru, Kyoto-shi, Kyoto 602-0008 (JP); Komori, Mariko, Kyoto-shi, Kyoto 602-0008 (JP)
(74) Representative: Jones, Elizabeth Louise

(57) **Abstract**

The probe for detecting a polymorphism in the MDR1 gene according to the present invention is a fluorescently labeled oligonucleotide selected from the group consisting of a P1 oligonucleotide and a P1' oligonucleotide.

SEQUENCE LISTING

<110>  ARKRAY, Inc.

<120>  Probe, polymorphism detection method, method of evaluating drug
efficacy or tolerance, and reagent kit

<130>  41.13.114279

<150>  JP 2011-219336
<151>  2011-10-03

<150>  JP 2012-190186
<151>  2012-08-30

<160>  21

<170>  PatentIn version 3.4

<210>  1
<211>  801
<212>  DNA
<213>  Artificial Sequence

<220>
<223>  Exon 12 of MDR1 gene having a polymorphism at the 1236th base of exon
12


<220>
<221>  misc_feature
<222>  (401)..(401)
<223>  y is c or t.

<400>  1
ttgtccagtt aagtcaattt ctttgtcact ttatccagct ctccacaaaa tatcactaaa      60

agtagttatt gtaacctagt aatctcttaa aatttgattc tgtttagaag ccaagtattg     120

acagctattc gaagagtggg cacaaaccag ataatattaa gggaaatttg gaattcagaa     180

atgttcactt cagttaccca tctcgaaaag aagttaaggt acagtgataa atgattaatc     240

aacaattaat ctattgaatg aagagtttct gatgttttct tgtagagatt ataaaaaagt     300

gcatgtatat ttaaacctag tgaacagtca gttcctatat cctgtgtctg tgaattgcct     360

tgaagttttt ttctcactcg tcctggtaga tcttgaaggg yctgaacctg aaggtgcaga     420

gtgggcagac ggtggccctg gttggaaaca gtggctgtgg gaagagcaca acagtccagc     480

tgatgcagag gctctatgac cccacagagg ggatggtgag atgacccatg cgagctagac     540

cctgcggtga tcagcagtca cattgcacat ctttctgatg ttgccctttc aattacaaat     600

gtatgaaagt cacacttact ttttattcca ggtcagtgtt gatggacagg atattaggac     660

cataaatgta aggtttctac gggaaatcat tggtgtggtg agtcaggaac ctgtattgtt     720

tgccaccacg atagctgaaa acattcgcta tggccgtgaa aatgtcacca tggatgagat     780

tgagaaagct gtcaaggaag c                                               801


<210>  2

<211>  18
<212>  DNA
<213>  Artificial Sequence

<220>
<223>  Probe

<400>  2
ttcaggttca gacccttc                                                    18


<210>  3
<211>  23
<212>  DNA
<213>  Artificial Sequence

<220>
<223>  Probe

<400>  3
gcaccttcag gttcagaccc ttc                                              23


<210>  4
<211>  28
<212>  DNA
<213>  Artificial Sequence

<220>
<223>  Probe

<400>  4
actctgcacc ttcaggttca gacccttc                                         28


<210>  5
<211>  38
<212>  DNA
<213>  Artificial Sequence

<220>
<223>  Probe

<400>  5
ccgtctgccc actctgcacc ttcaggttca gacccttc                              38


<210>  6
<211>  48
<212>  DNA
<213>  Artificial Sequence

<220>
<223>  Probe

<400>  6
accagggcca ccgtctgccc actctgcacc ttcaggttca gacccttc                   48


<210>  7
<211>  28
<212>  DNA
<213>  Artificial Sequence

<220>

<223>  Primer

<400>  7
ccttgaagtt tttttctcac tcgtcctg                                          28


<210>  8
<211>  21
<212>  DNA
<213>  Artificial Sequence

<220>
<223>  Primer

<400>  8
gtctgcccac tctgcacctt c                                                 21


<210>  9
<211>  19
<212>  DNA
<213>  Artificial Sequence

<220>
<223>  Probe

<400>  9
ctgccctcac aatctcttc                                                    19


<210>  10
<211>  18
<212>  DNA
<213>  Artificial Sequence

<220>
<223>  Probe

<400>  10
cccagaacct tctagttc                                                     18


<210>  11
<211>  23
<212>  DNA
<213>  Artificial Sequence

<220>
<223>  Primer

<400>  11
aaatgttgtc tggacaagca ctg                                               23


<210>  12
<211>  30
<212>  DNA
<213>  Artificial Sequence

<220>
<223>  Primer

<400>  12
aattaatcaa tcatatttag tttgactcac                                        30

<210>  13
<211>  20
<212>  DNA
<213>  Artificial Sequence

<220>
<223>  Primer

<400>  13
actgcagcat tgctgagaac                                                   20


<210>  14
<211>  21
<212>  DNA
<213>  Artificial Sequence

<220>
<223>  Primer

<400>  14
cagagaggct gccacatgct c                                                 21


<210>  15
<211>  511
<212>  DNA
<213>  Artificial Sequence

<220>
<223>  Exon 26 of MDR1 gene having a polymorphism at the 3435th base of exon
26


<220>
<221>  misc_feature
<222>  (256)..(256)
<223>  y is c or t.

<400>  15
ctcacagtaa cttggcagtt tcagtgtaag aaataatgat gttaattgtg ctacattcaa      60

agtgtgctgg tcctgaagtt gatctgtgaa ctcttgtttt cagctgcttg atggcaaaga     120

aataaagcga ctgaatgttc agtggctccg agcacacctg ggcatcgtgt cccaggagcc     180

catcctgttt gactgcagca ttgctgagaa cattgcctat ggagacaaca gccgggtggt     240

gtcacaggaa gagatygtga gggcagcaaa ggaggccaac atacatgcct tcatcgagtc     300

actgcctaat gtaagtctct cttcaaataa acagcctggg agcatgtggc agcctctctg     360

gcctatagtt tgatttataa ggggctggtt tcccagaagt gaagagaaat tagcaaccaa     420

atcacaccct tacctgtata caagcatctg gccacacttc ctgtttgggt tagttgttac     480

ctttacctga tcacctgacc ctccttgtga g                                    511


<210>  16
<211>  600
<212>  DNA
<213>  Artificial Sequence

<220>
<223>  Exon 21 of MDR1 gene having a polymorphism at the 2677th base of exon
21

<220>
<221>  misc_feature
<222>  (300)..(300)
<223>  d is a, t or g.

<400>  16
taaaataatg aatatagtct catgaaggtg agttttcaga aaatagaagc atgagttgtg      60

aagataatat ttttaaaatt tctctaattt gttttgtttt gcaggctrta ggttccaggc     120

ttgctgtaat tacccagaat atagcaaatc ttgggacagg aataattata tccttcatct     180

atggttggca actaacactg ttactcttag caattgtacc catcattgca atagcaggag     240

ttgttgaaat gaaaatgttg tctggacaag cactgaaaga taagaaagaa ctagaaggtd     300

ctgggaaggt gagtcaaact aaatatgatt gattaattaa gtagagtaaa gtattcyaat     360

cagtgttatt ttgtyactcc ctactgctta ctatgctcta agaatgtgtt tataaccatt     420

cctcaaagca atctttttca tgcttattca gtaaattaga aacttacaga aagtagcaaa     480

gccagttctt ggactcaaaa actgataatt aactttaaca gactttttca gttttcaggc     540

cattgtcttc acactgttct tccttcctcc ccactttcct ccttccctta gttatkttct     600

<210>  17
<211>  15
<212>  DNA
<213>  Artificial Sequence

<220>
<223>  Probe

<400>  17
tgaagggtct gaacc                                                        15

<210>  18
<211>  25
<212>  DNA
<213>  Artificial Sequence

<220>
<223>  Template oligo sequence

<400>  18
gatcttgaag ggtctgaacc tgaag                                             25

<210>  19
<211>  25
<212>  DNA
<213>  Artificial Sequence

<220>
<223>  Template oligo sequence

<400>  19

gatcttgaag ggcctgaacc tgaag                                              25

<210>  20
<211>  25
<212>  DNA
<213>  Artificial Sequence

<220>
<223>  Template oligo sequence

<400>  20
cttcaggttc agacccttca agatc                                              25

<210>  21
<211>  25
<212>  DNA
<213>  Artificial Sequence

<220>
<223>  Template oligo sequence

<400>  21
cttcaggttc aggcccttca agatc                                              25

## Description

### BACKGROUND

### Technical Field

The present invention relates to a probe for detecting a polymorphism, a method of detecting a polymorphism, a method of evaluating the efficacy of a drug, and a reagent kit for detecting a polymorphism.

### Related Art

A MDR1 gene is a gene encoding P-glycoprotein. P-glycoprotein is expressed in a variety of cells including cancer cells, hepatic cells, intestinal cells and cerebrovascular endothelial cells and plays a role in drug transport.
Further, a mutant version of the gene in which C of the 3435th base in exon 26 of the MDR1 gene is substituted with T (C3435T mutant-type), a mutant version of the gene in which G of the 2677th base in exon 21 of the MDR1 gene is substituted with A or T (G2677A/T mutant-type) and a mutant version of the gene in which C of the 1236th base in exon 12 of the MDR1 gene is substituted with T (C1236T mutant-type) are known to be useful as factors for estimating (or aiding) the prognosis of colon cancer (see, for example, Int. J. Colorectal. Dis., 2010, vol. 25, pp. 1167-1176 (Non-patent Document 1)).

As a method of measuring a gene polymorphism, a method is known in which, after performing PCR using primers designed to amplify a region containing a base to be measured, the resulting amplification product is cleaved with a restriction enzyme such that the presence or absence of cleavage depends on whether or not a mutation at the base exists and the resultant (restriction enzyme-treated product) is then subjected to electrophoresis to detect whether the amplification product has been cleaved or not (PCR-RFLP method).
As an alternative method, a method is also known in which, after amplifying a region containing a mutation by a PCR method, a nucleic acid probe labeled with a fluorescent dye is hybridized to a target nucleic acid and the decrease in the amount of light emission by the fluorescent dye is measured so as to analyze a mutant-type base sequence based on the results of melting curve analysis (see, for example, Japanese Patent Publication No. 3963422 (Patent Document 1)).

Further, it has been reported that the presence or absence of the C3435T mutant-type of the MDR1 gene can be easily detected with high sensitivity by using a specific nucleic acid probe (see, for example, Japanese Patent Publication No. 4454366 (Patent Document 2)).

In Non-patent Document 1, a PCR-RFLP method was employed as a method of detecting the C3435T mutant-type of the MDR1 gene. In a PCR-RFLP method, it is required that, after PCR is performed, the resulting amplification product be taken out and subjected to a treatment with a restriction enzyme. Therefore, the amplification product may contaminate the subsequent reaction systems, which leads to a false-positive or false-negative result in some cases. In addition, since a treatment with a restriction enzyme is performed after the completion of PCR and the resultant restriction enzyme-treated product is then subjected to electrophoresis, the time required for detection may become extremely long as well. Furthermore, since the operations (steps) of this method are complex, automation thereof is difficult.
Further, in Non-patent Document 1, as a method of detecting the G2677A/T mutant-type and the C1236T mutant-type of the MDR1 gene, a sequencing method is employed. Such a detection method utilizing sequence analysis requires labour and cost since, for example, after performing PCR, it is necessary to perform sequencing reactions and then electrophoresis using a sequencer. In addition, since it is required to process the resulting amplification product, the amplification product may contaminate the subsequent reaction systems.

In Patent Document 1, a polymorphism is detected by a method utilizing a nucleic acid probe. However, with regard to the design of the nucleic acid probe, the disclosure offered in Patent Document 1 is merely that the nucleic acid probe is designed in such a manner that, when a quenching probe whose terminus is labeled with a fluorescent dye is hybridized to its target sequence, at least one G-C pair is formed by the multiple base pairs of the hybrid which is generated between the nucleic acid probe and the target sequence at the terminal. Therefore, in the method described in Patent Document 1, it is required to use a nucleic acid probe having an appropriate sequence for each mutant type.
Furthermore, investigation by the present inventors revealed that the quenching probe does not function sufficiently when the GC content is excessively high in the sequence of the nucleic acid probe.

In Patent Document 2, there is disclosed a method of detecting C3435T mutation in exon 26 of the MDR1 gene; however, the G2677A/T mutant-type and C1236T mutant-type of the MDR1 gene cannot be detected with the nucleic acid probe described in Patent Document 2.
In view of these present circumstances, a further technical development for detecting a polymorphism in bases of the MDR1 gene other than the C3435T mutant-type has been strongly desired.

### SUMMARY OF THE INVENTION

### PROBLEMS TO BE SOLVED BY THE INVENTION

An object of the present invention is to provide a polymorphism detection probe which enables a C1236T mutation in exon 12 of the MDR1 gene to be easily detected with high sensitivity; a polymorphism detection method utilizing the probe; a method of evaluating the efficacy of a drug; and a reagent kit for detecting a polymorphism.

### MEANS FOR SOLVING THE PROBLEM

The present inventors discovered that, by designing a quenching probe based on a specific region containing the C1236T mutation in exon 12 of the MDR1 gene, a C1236T mutation can be detected by performing melting curve analysis using the quenching probe. The present invention was achieved based on such a discovery.
The present invention is as follows.
<1> A probe for detecting a polymorphism in the MDR1 gene, which is a fluorescently labeled oligonucleotide selected from the group consisting of the following P1 and P1':
   (P1) an oligonucleotide having an identity of at least 80% to a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye; and
   (P1') an oligonucleotide which hybridizes under stringent conditions to the complementary strand of a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye.
<2> The probe according to <1>, which is at least one fluorescently labeled oligonucleotide selected from the group consisting of the following P1-1 and P1'-1:
   (P1-1) an oligonucleotide having an identity of at least 80% to a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye, the oligonucleotide recognizing a polymorphism at the 401 st base of SEQ ID NO:1; and
   (P1'-1) an oligonucleotide which hybridizes under stringent conditions to the complementary strand of a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye, the oligonucleotide recognizing a polymorphism at the 40 1 st base of SEQ ID NO:1.
<3> The probe according to <1> or <2>, wherein the above-described P1 fluorescently labeled oligonucleotide or P1' fluorescently labeled oligonucleotide has the base corresponding to the 395th base labeled with a fluorescent dye at any one of the first to the third positions from the 3'-end.
<4> The probe according to any one of <1> to <3>, wherein the above-described P1 fluorescently labeled oligonucleotide or P1' fluorescently labeled oligonucleotide has the base corresponding to the 395th base labeled with a fluorescent dye at the 3'-end.
<5> The probe according to any one of <1> to <4>, wherein the above-described P1 fluorescently labeled oligonucleotide or P1' fluorescently labeled oligonucleotide emits fluorescence when it is not hybridized to a target sequence, and the fluorescence intensity of the P1 fluorescently labeled oligonucleotide or P1' fluorescently labeled oligonucleotide when hybridized to the target sequence is decreased or increased as compared to when not hybridized to the target sequence.
<6> The probe according to <5>, wherein the above-described P1 fluorescently labeled oligonucleotide or P1' fluorescently labeled oligonucleotide emits fluorescence when it is not hybridized to a target sequence, and the fluorescence intensity of the P1 fluorescently labeled oligonucleotide or P1' fluorescently labeled oligonucleotide when hybridized to the target sequence is decreased as compared to when not hybridized to the target sequence.
<7> The probe according to any one of <1> to <6>, wherein the above-described P1 fluorescently labeled oligonucleotide or P1' fluorescently labeled oligonucleotide has a length of 7 to 28 bases.
<8> The probe according to any one of <1> to <7>, wherein the above-described P1 fluorescently labeled oligonucleotide or P1' fluorescently labeled oligonucleotide has a length of 7 to 18 bases.
<9> The probe according to any one of <1> to <8>, which is a probe for melting curve analysis. Alternatively expressed, use of a probe according to any one of <1> to <8> for melting curve analysis.
<10> A method of detecting a polymorphism in the MDR1 gene, the method comprising the steps of:
   (I) bringing the probe according to any one of <1> to <9> into contact with a single-stranded nucleic acid contained in a sample to hybridize the above-described fluorescently labeled oligonucleotide to the above-described single-stranded nucleic acid, thereby obtaining a hybrid;
   (II) dissociating the above-described hybrid by changing the temperature of the sample containing the hybrid to measure the change in fluorescence signal caused by dissociation of the hybrid;
   (III) determining the Tm value, which is the dissociation temperature of the hybrid, based on the above-described change in fluorescence signal; and
   (IV) based on the above-described Tm value, detecting the presence of a polymorphism of the MDR1 gene in the above-described single-stranded nucleic acid in the above-described sample.
<11> The method according to <10>, which further includes the step of amplifying the nucleic acid prior to or simultaneously with the above-described step (I) of obtaining a hybrid.
<12> The method according to any one of <10> or <11>, which further includes the step of detecting, in the same system, at least either (one) of a polymorphism corresponding to the 256th base of the base sequence indicated in SEQ ID NO:15 or a polymorphism corresponding to the 300th base of the base sequence indicated in SEQ ID NO:16.
<13> The method according to any one of <10> to <12>, which includes the step of detecting, in the same system, a polymorphism corresponding to the 401 st base of the base sequence indicated in SEQ ID NO:1, a polymorphism corresponding to the 256th base of the base sequence indicated in SEQ ID NO:15 and a polymorphism corresponding to the 300th base of the base sequence indicated in SEQ ID NO:16.
<14> A method of evaluating efficacy of a drug, which includes the steps of:
   detecting a polymorphism in the MDR1 gene by the method of detecting a polymorphism according to any one of <10> to <13>; and
   determining (or predicting) tolerance to the drug or the efficacy of the drug based on the presence or absence of a detected polymorphism. Said method may be performed on a sample from a subject (e.g. a human) in relation to whom the evaluation is to be made. A sample (as used herein) comprises nucleic acid (e.g. DNA) which may contain said polymorphism.
<15> A reagent kit for detecting a polymorphism in the MDR1 gene, which includes the probe according to any one of <1> to <9>.
<16> The reagent kit according to <15>, which further includes a primer for amplifying a base sequence containing a region to which the above-described probe hybridizes.
<17> The reagent kit according to <15> or <16>, which further includes a probe for detecting a polymorphism corresponding to the 256th base of the base sequence indicated in SEQ ID NO:1 and a probe for detecting a polymorphism corresponding to the 300th base of the base sequence indicated in SEQ ID NO:16.
The invention also extends to the use of the reagent kit according to <15> to <17> or probe according to <1> to <9> for detecting said polymorphism in the MDR1 gene.

### EFFECTS OF THE INVENTION

According to the present invention, a polymorphism detection probe which allows a C1236T mutation in exon 12 of the MDR1 gene to be easily detected with high sensitivity; a polymorphism detection method utilizing the probe; a method of evaluating the efficacy of a drug; and a reagent kit for detecting a polymorphism are provided.

### BRIEF DESCRIPTION OF THE DRAWINGS

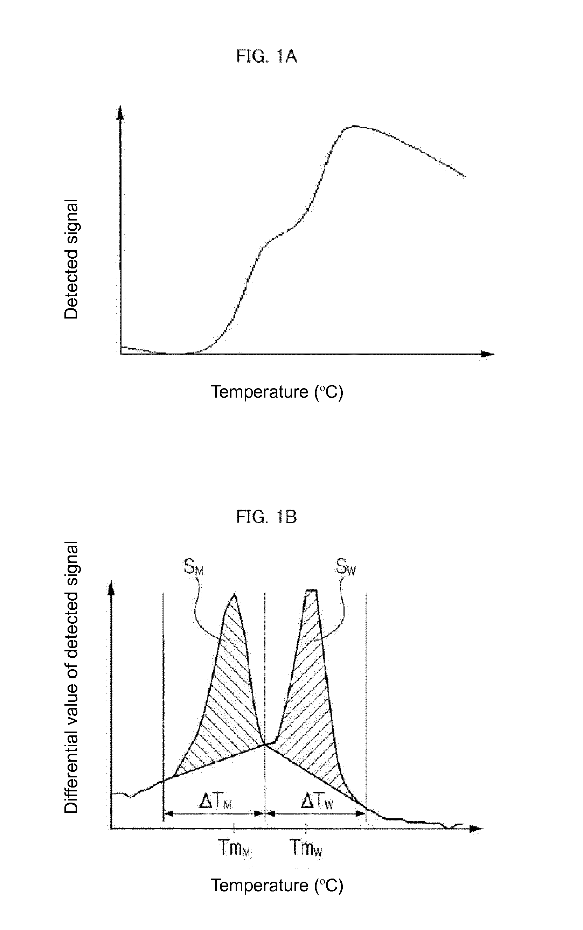

Fig. 1A is an example of a melting curve of a nucleic acid mixture, and Fig. 1B is an example of a differential melting curve of a nucleic acid mixture.
Fig. 2 depicts differential melting curves of samples according to Example 1 of the present invention.
Fig. 3A to Fig. 3F are differential melting curves of samples according to Example 2 of the present invention.
Fig. 4 depicts differential melting curves of samples according to Comparative Example 1 of the present invention.

### DETAILED DESCRIPTION OF THE INVENTION

The probe for detecting a polymorphism in the MDR1 gene according to the present invention (hereinafter also simply referred to as "the probe") is a probe for detecting a polymorphism in the MDR1 gene, the probe being at least one fluorescently labeled oligonucleotide selected from the group consisting of a P1 fluorescently labeled oligonucleotide and a P1' fluorescently labeled oligonucleotide:
(P1) an oligonucleotide having an identity of at least 80% to a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye; and
(P1') an oligonucleotide which hybridizes under stringent conditions to the complementary strand of a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye.
The method of detecting a polymorphism in the MDR1 gene according to the present invention is a method which includes detecting a polymorphism in the MDR1 gene using at least one probe for detecting a polymorphism in the MDR1 gene as described above.
The method of evaluating the efficacy of a drug according to the present invention is a method which includes detecting a polymorphism in the MDR1 gene by the above-described method of detecting a polymorphism in the MDR1 gene, and evaluating (e.g. determining or predicting) tolerance to a drug or the efficacy of a drug based on the detected presence or absence of the polymorphism.
The reagent kit for detecting a polymorphism according to the present invention is a kit which contains the probe for detecting a polymorphism in the MDR1 gene.

The "MDR1 gene" in the present invention is already known, and the base sequence thereof corresponds to the 4926th to the 214386th bases of Gene ID: 5243, NCBI Accession No. NG_011513. It is noted here that, in the present specification, unless otherwise specified, "the MDR1 gene" means the base sequence corresponding to exon 12 of the MDR1 gene indicated in SEQ ID NO:1.
The base sequence of SEQ ID NO:1 is the sequence of the 1st to the 80 1 st bases of NCBI dbSNP Accession NO. rs1128503.

In the present invention, the description of the base sequences of the sample nucleic acid to be detected in a sample and the polymorphism detection probe or primer shall also apply to complementary base sequences thereof, respectively, unless otherwise specified. Further, when the description of a particular base sequence is applied to a complementary base sequence thereof, the descriptions of base sequences recognized by the particular base sequence in the present invention should be applied provided that the recognition by the particular base sequence is replaced with recognition by a complementary base sequence of the particular base sequence, within the range of the common general technical knowledge of those skilled in the art.

In the present invention, the term "Tm value" is defined as the temperature at which a double-stranded nucleic acid dissociates (dissociation temperature: Tm), and is generally defined as the temperature at which the absorbance at 260 nm has increased by 50% of the total increase in absorbance resulting from complete dissociation of the double-stranded nucleic acid. More specifically, when a solution containing a double-stranded nucleic acid such as a double-stranded DNA is heated, the absorbance at 260 nm of the double-stranded nucleic acid gradually increases. This is because the hydrogen bonds between both strands of the double-stranded DNA are broken by heating, thereby dissociating the double-stranded DNA into single-stranded DNA (melting of DNA). When the double-stranded DNA has completely dissociated into single-stranded DNA, the single-stranded DNA exhibit an absorbance that is about 1.5 times the absorbance at the time of the initiation of the heating (i.e., the absorbance when the entire DNA is in the form of a double-stranded DNA), which serves as an indicator of the completion of the melting. The Tm value is defined based on this phenomenon.
In the present invention, when the phrase "the first to third bases from the 3' end" is used in connection to an oligonucleotide sequence, it is assumed that the base at the 3' end of the oligonucleotide chain is the first base from the 3' end. Similarly, when the phrase "the first to third bases from the 5' end" is used in connection to an oligonucleotide sequence, it is assumed that the base at the 5' end of the oligonucleotide chain is the first base from the 5' end.

In the present specification, the scope of the term "process" or "step" includes not only a discrete process/step, but also a process/step that cannot be clearly distinguished from another process/step as long as the expected effect of the process/step of interest is achieved. In the present specification, any numerical range expressed using "to" refers to a range including the numerical values before and after "to" as the minimum and maximum values, respectively.
In the case in which the amount of a component that may be included in the composition is indicated in the present invention, when there are multiple substances corresponding to the component in the composition, the indicated amount means the total amount of the multiple substances present in the composition, unless specifically stated otherwise.
In the present specification, the term "mutation" refers to a base sequence newly produced by substitution, deletion, overlapping of or insertion into a part of a wild-type base sequence. Further, the term "polymorphism" refers to a polymorphism of a base sequence caused by a mutation.
The present invention is described below.

### <Probe for Detecting Polymorphism in MDR1 Gene>

The probe for detecting a polymorphism in the MDR1 gene according to the present invention (hereinafter also simply referred to as "the probe") is a probe for detecting a polymorphism in the MDR1 gene, the probe including one fluorescently labeled oligonucleotide selected from the group consisting of a P1 fluorescently labeled oligonucleotide and a P1' fluorescently labeled oligonucleotide:
(P1) an oligonucleotide having an identity of at least 80% to a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye; and
(P1') an oligonucleotide which hybridizes under stringent conditions to the complementary strand of a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye. In other words, the P1' fluorescently labeled oligonucleotide of the present invention hybridizes, under stringent conditions, to a base sequence having a length of 7 to 38 bases including the 395th to the 40 1 st bases of the base sequence indicated in SEQ ID NO:1.

In the present invention, the above-described P1 and P1' fluorescently labeled oligonucleotides are probes capable of detecting a polymorphism at the 40 1 st base of the base sequence indicated in SEQ ID NO:1.

In the wild-type MDR1 gene, the base corresponding to the 40 1 st base of the sequence indicated in SEQ ID NO:1 is C (cytosine); however, in a mutant-type, the C is mutated to T (thymine) (hereinafter, also referred to as "the C1236T mutant-type") and this base corresponds to the 87296217th base of the 87133179th to the 87342639th bases of the MDR1 gene.

In the present invention, the above-described P1 and P1' fluorescently labeled oligonucleotides are base sequences complementary to a base sequence including the 395th to the 401 st bases of SEQ ID NO:1.

Specifically, in the present invention, a fluorescently labeled oligonucleotide which has homology has an identity of at least 80% to a sequence complementary to a base sequence of 7 to 38 bases in length including the 395th to the 401st bases of the base sequence indicated in SEQ ID NO:1. Here, the base corresponding to the 395th base is a cytosine. Further, from the standpoint of the detection sensitivity, the P1 fluorescently labeled oligonucleotide may also exhibit an identity of not less than 85%, not less than 90%, not less than 95%, not less than 96%, not less than 97%, not less than 98% or not less than 99% to a sequence complementary to a base sequence of 7 to 38 bases in length including the 395th to the 401st bases of the base sequence indicated in SEQ ID NO:1. As referred to herein, said 7 to 38 bases are consecutive bases of SEQ ID NO:1 which include the 395th to 401st bases of the base sequence.
When the identity between the above-described P1 fluorescently labeled oligonucleotide of the present invention and the sequence complementary to the base sequence having the same bases as SEQ ID NO:1 except that the base corresponding to the 395th base is C (cytosine) is less than 80%, the detection sensitivity for a sample nucleic acid containing a mutant-type MDR1 gene becomes low.

In the present invention, the above-described P1' fluorescently labeled oligonucleotide hybridizes under stringent conditions to the complementary strand of a sequence complementary to a base sequence of 7 to 38 bases in length including the 395th to the 401st bases of the base sequence indicated in SEQ ID NO:1. Here, the base corresponding to the 395th base is a cytosine.

The hybridization may be carried out (under stringent conditions) according to a known method or a method corresponding thereto, such as a method as described in Molecular Cloning 3rd (J. Sambrook et al., Cold Spring Harbor Lab. Press, 2001). This document is incorporated herein by reference.
The term "stringent conditions" means conditions in which specific hybrids are formed, but non-specific hybrids are not formed. Typical examples of the stringent conditions include, for example, conditions in which the hybridization is carried out at a potassium concentration from about 25 mM (e.g. 25 mM) to about 50 mM (e.g. 50 mM) and a magnesium concentration from about 1.0 mM (e.g. 1.0 mM) to about 5.0 mM (e.g. 5.0 mM). One example of the conditions of the present invention is conditions in which the hybridization is carried out in Tris-HCl (pH 8.6), 25 mM KCl, and 1.5 mM MgCl₂, but examples of the conditions of the present invention are not limited thereto. Other examples of the stringent conditions are described in Molecular Cloning 3rd (J. Sambrook et al., Cold Spring Harbor Lab. Press, 2001). This document is incorporated herein by reference. Those skilled in the art may readily choose such conditions by changing the hybridization reaction and/or the salt concentration of the hybridization reaction solution.

Furthermore, the P1 or P1' fluorescently labeled oligonucleotide of the present invention encompasses a fluorescently labeled oligonucleotide having a sequence wherein a base(s) has been inserted to, deleted from and/or substituted in the P1 or P1' fluorescently labeled oligonucleotide.
The fluorescently labeled oligonucleotide having a sequence wherein a base(s) has been inserted, deleted and/or substituted is not particularly limited, as long as the oligonucleotide exhibits an effect similar to that of the P1 or P1' fluorescently labeled oligonucleotide; and, in cases where a base(s) has been inserted, deleted and/or substituted, the position(s) of the insertion(s), deletion(s) and/or substitution(s) is not particularly limited. The number of bases that have been inserted, deleted and/or substituted may be, for example, 1 base, or 2 or more bases, such as from 1 base to 10 bases and from 1 base to 5 bases, although varying depending on the total length of the fluorescently labeled oligonucleotide.

The oligonucleotides in the above-described fluorescently labeled oligonucleotide also encompass oligonucleotides as well as modified oligonucleotides.
Examples of a structural unit of the above-described oligonucleotide include ribonucleotides, deoxyribonucleotides and artificial nucleic acids. Examples of the artificial nucleic acids include DNAs, RNAs, LNAs (Locked Nucleic Acids) which are RNA analogues, PNAs (Peptide Nucleic Acids) which are peptide nucleic acids, BNAs (Bridged Nucleic Acids) which are cross-linked nucleic acids, and the like.
The above-described oligonucleotides may be constituted by one or multiple types of the structural units described in the above.

The P1 or P1' fluorescently labeled oligonucleotide of the present invention needs to have a length from 7 mer to 38 mer. When the P1 or P1' fluorescently labeled oligonucleotide has a length of 6 mer or less or a length of 39 mer or more, the sensitivity for detecting a polymorphism in the MDR1 gene will be decreased.
In addition, the P1 or P1' fluorescently labeled oligonucleotide of the present invention may have a length from 7 mer to 28 mer, or a length from 7 mer to 18 mer. By using the range from 7 mer to 28 mer, for example, the detection sensitivity tends to be higher.
By varying the base length of the P1 or P1' fluorescently labeled oligonucleotide, for example, the Tm value, which is the dissociation temperature of a hybrid formed by the P1 or P1' fluorescently labeled oligonucleotide and its complementary strand (target sequence), may be adjusted to a desired value.

Examples of the base sequence of the P1 or P1' fluorescently labeled oligonucleotide of the present invention are shown in Table 1 below, but the present invention is not limited to these.
It is noted here that, in Table 1, the base corresponding to the 401st base of SEQ ID NO:1 is indicated with a capital letter. In addition, Table 1 also shows the Tm values of hybrids formed between the respective sample nucleic acids having the same bases as SEQ ID NO:1 except that the base corresponding to the 40 1 st base is T or C and the P1 fluorescently labeled oligonucleotide.
The Tm values were calculated using MeltCalc© 99 FREE (http://www.meltcalc.com/) and under the set conditions of: Oligoconc. [µM] of 0.2 and Na eq. [mM] of 50.

**[Table 1]**

| Sequence (5'→3') | Length (mer) | Tm value | | Δ value between C and T | SEQ ID NO : |
|---|---|---|---|---|---|
| | | T | C | | |
| Acccttc | 7 | 2.8 | -11.9 | 14.7 | - |
| ttcaggttcagAcccttc | 18 | 50 | 41 | 9 | 2 |
| gcaccttcaggttcagAcccttc | 23 | 59 | 53.2 | 5.8 | 3 |
| actctgcaccttcaggttcagAcccttc | 28 | 63.1 | 58.7 | 4.4 | 4 |
| ccgtctgcccactctgcaccttcaggttcagAcccttc | 38 | 71.6 | 68.6 | 2.6 | 5 |
| accagggccaccgtctgcccactctgcaccttcaggttcagAcccttc | 48 | 76.3 | 74.5 | 1.8 | 6 |

In the present invention, the difference between the Tm value when the fluorescently labeled oligonucleotide is hybridized with a sample nucleic acid in which the base corresponding to the 40 1 st base of SEQ ID NO:1 is T and the Tm value when the fluorescently labeled oligonucleotide is hybridized with a sample nucleic acid in which the base corresponding to the 401st base of SEQ ID NO:1 is C is preferably not less than 1.5°C, more preferably not less than 2.0°C, still more preferably not less than 5°C, and yet still more preferably not less than 9.0°C. When the above-described difference in the Tm value is not less than 1.5°C, for example, a mutant-type of the 401st base in SEQ ID NO:1 can be detected with a higher sensitivity.

Examples of a method of increasing the difference in the Tm value include a method by which a probe is allowed to contain a base which mismatches with a base sequence corresponding to a region to which the probe hybridizes. Specific examples include those methods described in Nature Biotech (1997) vol. 15, pp. 331-335 and the like.

Further, the P1 or P1' fluorescently labeled oligonucleotide of the present invention needs to be labeled with a fluorescent dye at its base corresponding to the 395th base (cytosine).

In the P1 or P1' fluorescently labeled oligonucleotide, the fluorescently labeled base corresponding to the 395th base may exist at a position of any one of the 1 st to 3rd positions from the 3' end of the P1 or P1' fluorescently labeled oligonucleotide. Alternatively, the fluorescently labeled base may exist at the 3' end of the P1 or P1' fluorescently labeled oligonucleotide. Thereby, for example, the sensitivity for detecting a polymorphism is further improved. In addition, the P1 or P1' fluorescently labeled oligonucleotide may be obtained with good productivity (or efficacy).

The fluorescently labeled oligonucleotide of the present invention may be a fluorescently labeled oligonucleotide in which the fluorescence intensity at the time when the oligonucleotide is hybridized to its target sequence is decreased (quenched) or increased as compared to the fluorescence intensity at the time when the oligonucleotide is not hybridized to its target sequence. In particular, the fluorescently labeled oligonucleotide of the present invention may be a fluorescently labeled oligonucleotide in which the fluorescence intensity at the time when the oligonucleotide is hybridized to its target sequence is decreased as compared to the fluorescence intensity at the time when the oligonucleotide is not hybridized to its target sequence.

A probe that uses the "fluorescence quenching phenomenon" as described above is generally referred to as a guanine quenching probe, and it is known as Q PROBE^{®}. Among such probes, an oligonucleotide which has been designed so that its 3' or 5' end is a cytosine (C) and which has been labeled with a fluorescent dye so that the fluorescence emission is reduced when the C at the 3' or 5' end comes into proximity with a guanine (G) is especially preferable. By using such a probe, the hybridization and dissociation of the probe may be readily checked by the change in its signal.

A known detection method other than the detection method using a Q PROBE^{®} may also be applied. Examples of such a detection method include a TAQ-MAN probe method, a hybridization probe method, a molecular beacon method, and a MGB probe method.

The fluorescent dye is not particularly limited, and examples of the fluorescent dye include fluorescein, phosphor, rhodamine and polymethine dye derivatives. Examples of commercially available products of such fluorescent dyes include Pacific Blue, BODIPY FL, FluorePrime, Fluoredite, FAM, Cy3 and Cy5, and TAMRA.
The detection conditions of the fluorescently-labeled oligonucleotide are not particularly limited, and may be decided, as appropriate, in accordance with the fluorescent dye to be used. For example, Pacific Blue can be detected at a detection wavelength from 445 nm to 480 nm, TAMRA can be detected at a detection wavelength from 585 nm to 700 nm, and BODIPY FL can be detected at a detection wavelength from 520 nm to 555 nm.
By using a probe having such a fluorescent dye, hybridization and dissociation of the probe can be readily confirmed based on the change in fluorescence signal therefrom. Attachment of a fluorescent dye to the oligonucleotide may be carried out according to an ordinary method, such as a method described in JP-A No. 2002-119291.
It should be noted that, in the present invention, the same fluorescent dye may be used, or alternatively, different fluorescent dyes may be used to label one or more of the oligonucleotides.

In addition, the fluorescently-labeled oligonucleotide may have, for example, a phosphate group added to its 3' end. Addition of a phosphate group to the 3' end of the fluorescently-labeled oligonucleotide suppresses elongation of the probe itself by a gene amplification reaction. As described below, DNA in which the presence or absence of a mutation should be detected (target DNA) may be prepared using a gene amplification method such as PCR. When the fluorescently-labeled oligonucleotide that has a phosphate group added to its 3' end is used, the amplification reaction can be carried out even in the presence of the oligonucleotide in the reaction solution of the amplification reaction.
A similar effect can be obtained also by adding a labeling substance (a fluorescent dye) as described above to the 3' end.

The above-described P1 and P1' fluorescently labeled oligonucleotides may be used as a probe for detecting a polymorphism in the MDR1 gene, particularly the C1236T mutant-type of the MDR1 gene.
In addition, the probe for detecting a polymorphism in the MDR1 gene may be used as a probe for melting curve analysis.
The P1 fluorescently labeled oligonucleotide and P1' fluorescently labeled oligonucleotide according to the present invention may be produced according to a conventional method known as a method for synthesizing an oligonucleotide, such as a method as described in JP-ANo. 2002-119291, except that bases are used so that the base corresponding to the 395th base in the base sequence indicated in SEQ ID NO:1 is a cytosine and the base corresponding to the 395th base is labeled with a fluorescent dye.

More preferable embodiments of the P1 fluorescently labeled oligonucleotide and the P1' fluorescently labeled oligonucleotide include the following P1-1 fluorescently labeled oligonucleotide and the following P1'-1 fluorescently labeled oligonucleotide:
(P1-1) an oligonucleotide having an identity of at least 80% to a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye, the oligonucleotide recognizing a polymorphism at the 401 st base of SEQ ID NO:1; and
(P1'-1) an oligonucleotide which hybridizes under stringent conditions to the complementary strand of a sequence complementary to a base sequence of 7 to 38 bases in length including the 395th to the 401st bases of the base sequence indicated in SEQ ID NO:1, wherein the base corresponding to the 395th base is a cytosine labeled with a fluorescent dye, the oligonucleotide recognizing a polymorphism at the 40 1 st base of SEQ ID NO:1.
   It should be noted that "recognizing a polymorphism at the 401st base of SEQ ID NO:1" has the same meaning as "binding to the 401st base of SEQ ID NO:1, which exhibits a polymorphism".

### <Primer>

In the below-described method of detecting a polymorphism in the MDR1 gene, primers are used in amplifying a sequence having a MDR1 gene polymorphism to be detected by a PCR method.
The primer that may be used in the present invention is not particularly limited, as long as it is capable of amplifying a nucleic acid that contains the base corresponding to the 401 st base of the sequence indicated in SEQ ID NO:1, which is a desired MDR1 gene polymorphism site to be detected.

The primers used in the PCR method are not particularly restricted as long as they are capable of amplifying a base sequence containing a region to which the polymorphism detection probe according to the present invention hybridizes, and such primers can be designed as appropriate by those skilled in the art based on the base sequences indicated in SEQ ID NOs:1 to 6. The length and Tm value of the primer may be a length from 12 mer to 40 mer and a value from 40°C to 70°C, or a length from 16 mer to 30 mer and a value from 55°C to 60°C.
The lengths of individual primers in a primer set do not need to be the same, although the Tm values of these primers are preferably approximately the same (or the difference between the Tm values of these primers is preferably within 5°C).

Examples of the primers that may be used for amplifying a base sequence containing a region to which the polymorphism detection probe according to the present invention used in the polymorphism detection method according to the present invention hybridizes are shown below. It is noted here that the following examples are provided for illustrative purposes only and that, therefore, the present invention is not restricted thereto.

**[Table 2]**

| Primer | Sequence (5'→3') | Length (mer) | SEQ ID NO: |
|---|---|---|---|
| MDR1 (1236)F | ccttgaagtttttttctcactcgtcctg | 28 | 7 |
| MDR1 (1236)R | gtctgcccactctgcaccttc | 21 | 8 |

When amplifying a base sequence containing a region to which the polymorphism detection probe according to the present invention hybridizes, from the standpoint of sensitivity and efficiency, for example, the oligonucleotides shown in Table 2 may be used as a set of paired primers.

The method of detecting a polymorphism is not particularly limited, as long as it is a method in which a fluorescently labeled oligonucleotide as described above is used as a probe. As an example of the polymorphism detection method in which a fluorescently labeled oligonucleotide as described above is used as a probe, a method of detecting a polymorphism using Tm analysis is described below.

### <Polymorphism Detection Method>

The method of detecting a polymorphism in the MDR1 gene according to the present invention is a method of detecting a polymorphism in the MDR1 gene which includes detecting a polymorphism in the MDR1 gene by using at least one probe for detecting a polymorphism in the MDR1 gene as described above.
The method of detecting a polymorphism of the present invention may include at least one probe for detecting a polymorphism as described above, and this may make it possible to detect a polymorphism(s) in the MDR1 gene easily and with high sensitivity.
In addition, the method of detecting a polymorphism according to the present invention may be employed as a method of detecting a polymorphism in various human genes, and may include the below-described processes or steps (I) to (IV), and may include the below-described step (V). The method of detecting a polymorphism according to the present invention has the feature of using the above-described probe, and other configurations, conditions and the like are not particularly limited by the description below.

Step (I): contacting the fluorescently-labeled probe with a single-stranded nucleic acid in a sample, to obtain a hybrid.
Step (II): dissociating the hybrid by changing the temperature of the sample containing the hybrid, and measuring the change in fluorescence signal due to the dissociation of the hybrid.
Step (III): measuring the Tm value, which is the dissociation temperature of the hybrid, based on the change in fluorescence signal.
Step (IV): detecting the presence of the MDR1 polymorphism on the single-stranded nucleic acid in the sample, based on the Tm value.
Step (V): determining the abundance ratio of the single-stranded nucleic acid having the polymorphism in the total single-stranded nucleic acids contained in the sample, based on the presence of the polymorphism.

Furthermore, the method according to the present invention may further include amplifying the nucleic acid before obtaining the hybrid in step (I) or simultaneously with obtaining the hybrid in step (I), in addition to steps (I) to (IV) or in addition to steps (I) to (V). The measurement of the Tm value in step (III) may include not only measuring the dissociation temperature of the hybrid, but also measuring the differential values of the fluorescence signal that changes according to the temperature when the hybrid is melted.

In the present invention, the nucleic acid in the sample may be a single-stranded nucleic acid or a double-stranded nucleic acid. In the case in which the nucleic acid is a double-stranded nucleic acid, the method may include, for example, melting (dissociating) the double-stranded nucleic acid in the sample into single-stranded nucleic acids by heating before being hybridized with the fluorescently-labeled probe. The dissociation of a double-stranded nucleic acid into single-stranded nucleic acids enables hybridization with the fluorescently-labeled probe.

In the present invention, the nucleic acid contained in the sample to be detected may be, for example, a nucleic acid originally contained in a biological sample, or an amplification product obtained by amplifying a region of the gene of interest that contains a mutated site(s) of the MDR1 gene by PCR or the like using a nucleic acid originally contained in a biological sample as a template with a view to improving the detection accuracy. The length of the amplification product is not particularly limited, and may be, for example, a length from 50 mer to 1000 mer, or a length from 80 mer to 200 mer. Furthermore, the nucleic acid in the sample may be, for example, a cDNA that has been synthesized from RNAs derived from a biological sample (e.g., total RNAs, mRNAs, etc.) by RT-PCR (Reverse Transcription PCR).

In the present invention, the addition ratio (molar ratio) of the probe according to the present invention relative to the nucleic acids in the sample is not particularly limited. The amount of the probe to be added may be, for example, no more than 1 fold (by mol) the amount of DNA in the sample. From the viewpoint of ensuring a sufficient detection signal, the addition ratio of the probe according to the present invention to be added relative to the nucleic acids in the sample (in a molar ratio) may be 0.1 or lower.
The "nucleic acids in the sample" may be, for example, the total nucleic acids to be detected that have the polymorphism to be detected and nucleic acids, other than the nucleic acids to be detected, that do not have the polymorphism, or the total amplification products containing a detection target sequence having the polymorphism to be detected and amplification products containing a sequence, other than the detection target sequence, that does not have the polymorphism. Although the ratio of the nucleic acid to be detected relative to nucleic acids in the sample is usually unknown in advance, the consequent addition ratio of the probe relative to the nucleic acids to be detected (or the amplification products containing a sequence to be detected) (in a molar ratio) may be 10 or lower. The addition ratio of the probe relative to the nucleic acids to be detected (or the amplification products containing a sequence to be detected) (in a molar ratio) may be 5 or lower, or 3 or lower. The lower limit of the ratio is not particularly limited, and may be, for example, 0.001 or higher, 0.01 or higher, or 0.1 or higher.

The above-described addition ratio of the fluorescently-labeled probe according to the present invention relative to the DNA may be, for example, a molar ratio relative to the double-stranded nucleic acids or a molar ratio relative to the single-stranded nucleic acids.

In the present invention, the measurement of the change in the signal caused by a temperature change for determining a Tm value may be carried out by measuring the absorbance at 260 nm on the basis of the principle described above. However, the measurement may be carried out by measuring a signal which is based on a signal from the label attached to the fluorescently-labeled probe, and which varies in accordance with the degree of formation of a hybrid of a single-stranded DNA and the probe. Therefore, the above-described fluorescently-labeled oligonucleotide may be used as the fluorescently-labeled probe. Examples of the fluorescently-labeled oligonucleotide (hereinafter sometimes collectively referred to as "fluorescently-labeled oligonucleotide") include a fluorescently-labeled oligonucleotide in respect of which the fluorescence intensity when the oligonucleotide is hybridized with a target sequence thereof is decreased (quenched) as compared to the fluorescence intensity when the oligonucleotide is not hybridized with the target sequence thereof, and a fluorescently-labeled oligonucleotide in respect of which the fluorescence intensity when the oligonucleotide is hybridized with a target sequence thereof is increased as compared to the fluorescence intensity when the oligonucleotide is not hybridized with the target sequence thereof.
The former fluorescently-labeled oligonucleotide does not show a fluorescence signal or only a weak fluorescence signal when the fluorescently-labeled oligonucleotide forms a hybrid (a double-stranded DNA) with the sequence to be detected; however, the fluorescently-labeled oligonucleotide shows a fluorescence signal or shows an increased fluorescence signal when the ftuorescently-labeled oligonucleotide is dissociated by heating.
The latter fluorescently-labeled oligonucleotide shows a fluorescence signal when the fluorescently-labeled oligonucleotide forms a hybrid (a double-stranded DNA) with the sequence to be detected; however, the ftuorescently-labeled oligonucleotide shows a decreased fluorescence signal or ceases to show a fluorescent signal when the fluorescently-labeled oligonucleotide is dissociated by heating. Therefore, similar to the measurement of the absorbance at 260 nm described above, the progress of melting can be monitored, and the Tm value can be determined by detecting the change in fluorescence signal from the fluorescent label under the conditions specific to the fluorescent label (for example, the fluorescence wavelength thereof).

The method for detecting the change in signal based on a signal from the fluorescent dye in the polymorphism detection method according to the present invention is described below by way of specific examples. The polymorphism detection method according to the present invention has as a feature the use of the fluorescently-labeled polymorphism detection probe, and other processes and conditions of the method are not limited in any way.

The sample containing a nucleic acid that serves as a template for nucleic acid amplification is not particularly limited as long as the sample contains a nucleic acid, particularly the MDR1 gene. Examples of such a sample include a sample that is derived from or can be derived from any biological source, examples of which include: a tissue such as colon or lung; a hemocyte such as a leukocyte cell; whole blood; plasma; sputum; a suspension of oral mucosa; a somatic cell of nail, hair or the like; a germ cell; milk; ascitic fluid; a paraffin-embedded tissue; gastric juice; gastric lavage fluid; urine; peritoneal fluid; amniotic fluid; and a cell culture. The method for sampling the sample, the method for preparing the sample containing a nucleic acid, and the like are not limited, and, conventional methods known in the art may be employed therefor. A nucleic acid obtained from such a biological source may be directly used as the template, or may be used after the sample has been subjected to pretreatment that modifies the properties of the sample.
For example, in the case in which whole blood is used as the sample, the isolation of genomic DNA from the whole blood may be carried out by a conventional method known in the art. For example, a commercially available genomic DNA isolation kit (trade name: GFX GENOMIC BLOOD DNA PURIFICATION KIT, available from GE Healthcare Biosciences), etc. may be used.

Next, a fluorescently-labeled polymorphism detection probe including the fluorescently-labeled oligonucleotide is added to the sample containing an isolated genomic DNA.
The fluorescently-labeled probe may be added to a liquid sample containing the isolated genomic DNA, or may be mixed with the genomic DNA in an appropriate solvent. The solvent is not particularly limited, and examples of the solvent include conventional solvents known in the art, such as: a buffer solution such as Tris-HCl; a solvent containing at least one of KCl, MgCl₂, MgSO₄, or glycerol; and a PCR reaction solution.

The timing of the addition of the fluorescently-labeled probe is not particularly limited. For example, in the case in which an amplification process such as PCR described below is carried out, the fluorescently-labeled probe may be added to the PCR amplification products after the amplification process is carried out, or may be added before the amplification process is carried out.
In the case in which the fluorescently-labeled probe is added before an amplification process such as PCR is carried out, for example, a fluorescent dye or a phosphate group may have been added to the 3' end of the probe, as described above.

The method of amplifying a nucleic acid may be, for example, a method in which a polymerase is employed. Examples thereof include a PCR method, an ICAN method, a LAMP method, and an NASBA method. In the case in which the amplification is carried out by a method in which a polymerase is employed, the amplification may be carried out in the presence of the fluorescently-labeled probe according to the present invention. Those skilled in the art would be able to easily adjust the reaction conditions of the amplification and the like in accordance with the fluorescently-labeled probe and the polymerase to be used. In the case in which the amplification is carried out in the presence of the fluorescently-labeled probe according to the present invention, a polymorphism can be detected by analyzing the Tm value of the fluorescently-labeled probe only after the amplification of the nucleic acid is carried out, and, therefore, it is not necessary to separate the amplification product after completion of the reaction. Thus, contamination by (or of) the amplification product does not occur. In addition, since the detection can be carried out by the same apparatus as the apparatus required for the amplification, conveyance of a vessel is unnecessary, and automatization of the process is facilitated.

The DNA polymerase to be used in the PCR method may be selected, without particular limitation, from DNA polymerases that are usually used for PCR. Examples of the DNA polymerase include GENE TAQ (trade name, manufactured by NIPPON GENE CO., LTD.), PRIMESTAR MAX DNA POLYMERASE (trade name, manufactured by Takara Bio Inc.), and a Taq polymerase.
The amount of the polymerase to be used is not particularly limited as long as a usually-applied polymerase concentration is provided. For example, in the case in which a Taq polymerase is used, the concentration of the Taq polymerase may be, for example, a concentration from 0.01 U to 100 U relative to 50 µl of the reaction solution. In this range, for example, the sensitivity of the detection of the polymorphism in the MDR1 gene tends to be increased.

The PCR method may be carried out under the conditions appropriately selected from usually-employed conditions.
When the amplification is carried out, the amplification may be monitored using real-time PCR so that the copy number of the DNA (a sequence to be detected) contained in the sample can be measured. In other words, the proportion of probes forming hybrids is increased as the amplification of the DNA (a sequence to be detected) by PCR proceeds, thereby changing the fluorescence intensity. By monitoring the change in fluorescence intensity, the copy number and/or the abundance ratio of the sequence to be detected (either a normal DNA or a mutant DNA) contained in the sample can be obtained.

In the polymorphism detection method according to the present invention, the fluorescently-labeled oligonucleotide and a single-stranded nucleic acid in the sample are brought into contact with each other, thereby allowing hybridization to occur. The single-stranded nucleic acid in the sample can be prepared by, for example, dissociating the PCR amplification products obtained in the above-described manner.

The heating temperature employed for dissociation of the PCR amplification products (the heating temperature in the dissociation process) is not particularly limited as long as it is a temperature at which the amplification products can be dissociated. For example, the heating temperature may be in the range from 85°C to 95°C. The heating time is not particularly limited, either. The heating time may be, for example, in the range from 1 second to 10 minutes, or from 1 second to 5 minutes.

The hybridization of the dissociated single-stranded DNA and the fluorescently-labeled oligonucleotide may be carried out by, for example, decreasing, after the dissociation process, the temperature from the heating temperature employed in the dissociation process. The temperature condition for the hybridization may be, for example, in the range from 40°C to 50°C.

The volume and concentration of each component in the reaction solution in the hybridization process are not particularly limited. In regard to specific examples thereof, the concentration of DNA in the reaction solution may be, for example, a concentration from 0.01 µM to 1 µM, or a concentration from 0.1 µM to 0.5 µM. The concentration of the fluorescently-labeled oligonucleotide may be, for example, in a range in which the above-described addition ratio relative to the DNA is satisfied, and may be, for example, a concentration from 0.001 µM to 10 µM, or a concentration from 0.001 µM to 1 µM.

The resultant hybrid of the single-stranded DNA and the fluorescently-labeled oligonucleotide is gradually heated, and the change in fluorescence signal caused by the temperature increase is measured. For example, in the case of using Q PROBE^{®}, the fluorescence intensity when the probe is hybridized with the single-stranded DNA is decreased (or quenched) as compared to the fluorescence intensity in the dissociated state. Therefore, for example, the hybrid emitting decreased fluorescence or the quenched hybrid may be gradually heated, and the increase in fluorescence intensity caused by the temperature increase may be measured.

The temperature range in which the change in fluorescence intensity is measured is not particularly limited, and the initial temperature may be, for example, a temperature from room temperature to 85°C, or a temperature from 25°C to 70°C. The final temperature may be, for example, a temperature from 40°C to 105°C. The rate of temperature increase is not particularly limited, either, and may be, for example, in the range from 0.1°C/sec to 20°C/sec, or in the range from 0.3°C/sec to 5°C/sec.

Next, the change in signal is analyzed to determine the Tm value. More specifically, the Tm value may be determined by calculating a differential value at each temperature (-d(Fluorescence Intensity)/dt) from the fluorescence intensity obtained, and taking the temperature at which the differential value has the lowest value as the Tm value. The Tm value may alternatively be determined as the point at which the increase in fluorescence intensity per unit time ((Increase in Fluorescence Intensity)/t) has the largest value. On the contrary, in the case in which a probe in relation to which the signal intensity is increased by the formation of the hybrid, rather than a quenching probe, is used as the fluorescently-labeled probe, the signal analysis and the determination of the Tm value may be carried out by measuring the decrease in fluorescence intensity.

In the present invention, the change in fluorescence signal caused by the temperature increase (preferably an increase in fluorescence intensity) may be measured while heating the hybrid as described above. However, instead of this method, the measurement of the change in signal may alternatively be carried out, for example, during the course of hybrid formation. In other words, the temperature of the sample, to which the probe has been added, may be decreased, and the change in fluorescence signal caused by the temperature decrease may be measured during the course of hybrid formation.

For example, in the case in which Q PROBE^{®} is used, the fluorescence intensity is high when the probe is added to the sample since the probe is in the dissociated state. However, when the hybrid is formed by a decrease in temperature, the fluorescence is decreased (or quenched). Therefore, for example, a decrease in fluorescence intensity caused by the temperature decrease may be measured while gradually decreasing the temperature of the heated sample.
On the other hand, in the case in which a probe in relation to which the signal therefrom is increased by hybrid formation is used, the fluorescence intensity is low (or quenched) when the probe is added to the sample since the probe is in the dissociated state. However, when the hybrid is formed by a decrease in temperature, the fluorescence intensity is increased. Therefore, for example, the increase in fluorescence intensity caused by the temperature decrease may be measured while gradually decreasing the temperature of the sample.

Here, the polymorphism detection method according to the present invention includes detecting, in addition to a mutant-type MDR1 gene having a mutation at the 1236th base of exon 12 (the 401st base of the base sequence indicated in SEQ ID NO:1), at least one of a mutant-type MDR1 gene having a mutation at the 3435th base of exon 26 (the 256th base of the base sequence indicated in SEQ ID NO:15) or a mutant-type MDR1 gene having a mutation at the 2677th base of exon 21 (the 300th base of the base sequence indicated in SEQ ID NO:16).

By this, in addition to the mutant-type of the 401 st base of the base sequence indicated in SEQ ID NO:1, at least one of the mutant-type of the 256th base of the base sequence indicated in SEQ ID NO:15 or the mutant-type of the 300th base of the base sequence indicated in SEQ ID NO:16 can be further detected.

Specific means for the detection are not particularly restricted and examples thereof include the use of the probe according to the present invention which detects a polymorphism at the 40 1 st base of the base sequence indicated in SEQ ID NO:1 in combination with at least one of a probe which detects a polymorphism corresponding to the 256th base of the base sequence indicated in SEQ ID NO:15 or a probe which detects a polymorphism corresponding to the 300th base of the base sequence indicated in SEQ ID NO:16.

The above-described probe which detects a polymorphism corresponding to the 256th base of the base sequence indicated in SEQ ID NO:15 is not particularly restricted and examples thereof include the probe described in JP-A No. 2005-287335 and a probe having the base sequence indicated in SEQ ID NO:9.

The above-described probe which detects a polymorphism corresponding to the 300th base of the base sequence indicated in SEQ ID NO:16 is not particularly restricted and examples thereof include a probe having the base sequence indicated in SEQ ID NO:10.

Further, the polymorphism detection method according to the present invention also includes the process of detecting, in addition to a polymorphism at the 401 st base of the base sequence indicated in SEQ ID NO:1, at least one of a polymorphism at the 256th base of the base sequence indicated in SEQ ID NO:15 or a polymorphism at the 300th base of the base sequence indicated in SEQ ID NO:16, in the same system.

By this, in addition to a polymorphism at the 401st base of the base sequence indicated in SEQ ID NO:1, at least one of a polymorphism at the 256th base of the base sequence indicated in SEQ ID NO:15 or a polymorphism at the 300th base of the base sequence indicated in SEQ ID NO:16 can also be detected using the same system, which is more preferred from the standpoint of convenience.

The method of detecting multiple polymorphisms present on different exons in the same system is not particularly restricted. For example, the method may be a method in which individual probes capable of detecting these polymorphisms are preliminarily mixed and the resulting mixture is added to a sample, or a method in which individual probes capable of detecting these polymorphisms are consecutively or continuously added to a sample containing a single-stranded nucleic acid(s).
The term "system" means an independent reaction system formed with a sample containing a hybrid in which a fluorescently labeled oligonucleotide and a single-stranded nucleic acid are hybridized.

The polymorphism detection method according to the present invention may include the process of detecting, in the same system, a polymorphism corresponding to the 401 st base of the base sequence indicated in SEQ ID NO:1, a polymorphism corresponding to the 256th base of the base sequence indicated in SEQ ID NO:15 and a polymorphism corresponding to the 300th base of the base sequence indicated in SEQ ID NO:16.

By this, not only can the three types of gene polymorphism be easily detected in one system, but also the later-described drug tolerance and efficacy associated with these three types of polymorphism can be more accurately estimated.

Specific means for the detection are not particularly restricted and examples thereof include the use of three types of probes in combination, which are the probe according to the present invention which detects a polymorphism at the 401 st base of the base sequence indicated in SEQ ID NO:1, a probe which detects a polymorphism corresponding to the 256th base of the base sequence indicated in SEQ ID NO:15 and a probe which detects a polymorphism corresponding to the 300th base of the base sequence indicated in SEQ ID NO:16.
Here, with regard to the preferred embodiments and sequences of the respective probes, the above-described matters relating thereto are applicable.

### <Method of Evaluating Drug Efficacy or Tolerance>

The method of evaluating drug efficacy or tolerance of the present invention includes detecting a polymorphism in the MDR1 gene by the above-described polymorphism detection method, and evaluating the tolerance to a drug or the efficacy of a drug based on the results of the detection.
In the above-described polymorphism detection method, a polymorphism in the MDR1 gene may be detected with a high sensitivity and easily by using the probe of the present invention, and therefore, based on this polymorphism in the MDR1 gene, evaluation of a drug may be carried out with a high sensitivity and easily.

In addition, evaluation of tolerance to a drug and efficacy of a drug may be carried out based on whether a polymorphism(s) exists or not and/or based on the abundance ratio of a mutant sequence(s) and/or a normal sequence(s). The method of evaluating the efficacy of a drug of the present invention is useful in, for example, deciding whether the therapeutic strategy of a disease should be shifted so as to increase the dosage of the drug or use another therapeutic agent instead of the drug, based on whether a mutation(s) exists or not and/or based on the abundance ratio of a mutant sequence(s).
Further, specific examples of the drug to be evaluated include anticancer drugs, antihypertensive drugs, immunosuppressive agents and centrally acting drugs, and particularly include anticancer drugs.

### <Reagent Kit>

The reagent kit for detecting a polymorphism according to the present invention includes the above-described polymorphism detection probe(s).
Since this reagent kit for detecting a polymorphism includes at least one of the above-described polymorphism detection probes, it is able to, for example, more easily detect a polymorphism in the MDR1 gene.

In addition, the reagent kit of the present invention may further contain the above-described primer for amplifying a sequence having a MDR1 gene polymorphism to be detected. This enables the reagent kit of the present invention to detect a polymorphism in the MDR1 gene with good accuracy.
With regard to a probe(s) and primer(s) that may be contained in the reagent kit, the above descriptions may be applied as they are.

Further, the reagent kit for detecting a polymorphism according to the present invention may preferably further include, in addition to the probe for detecting a polymorphism in the MDR1 gene, at least one probe selected from the group consisting of the above-described probe which detects a polymorphism corresponding to the 256th base of the base sequence indicated in SEQ ID NO:15 and the probe which detects a polymorphism corresponding to the 300th base of the base sequence indicated in SEQ ID NO:16.
By allowing the reagent kit for detecting a polymorphism according to the present invention to further include the above-described other probes in addition to the polymorphism detection probe for the MDR1 gene, multiple polymorphisms present in multiple exons can be detected easily.

In the case in which two or more types of oligonucleotide are contained as the probes, the oligonucleotides may be contained as a mixture, or may be contained separately.
The two or more types of fluorescently-labeled oligonucleotide may be respectively labeled with fluorescent dyes having different emission wavelengths from each other.
By using probes labeled with respectively different fluorescent dyes, detection of the signal from each fluorescently-labeled oligonucleotide can simultaneously be carried out even in a single reaction system.

Besides the probe and the primers, the reagent kit according to the present invention may further include reagents required for carrying out nucleic acid amplification in the detection method according to the present invention. The probe, the primers and other reagents may be separately contained, or some of them may be contained as a mixture.
The term "separately contained" may refer to a state in which individual reagents are separated from each other such that the non-contact state therebetween is maintained, and does not necessarily require that the individual reagents be contained in separate containers that can be independently handled.
When the reagent kit includes a primer set for amplifying a base sequence including a base at the polymorphism site (a region to which the probe can hybridize), detection of the polymorphism with higher sensitivity, for example, can be achieved.

The reagent kit according to the present invention may further include an instruction manual that describes instructions for the generation of a differential melting curve for a sample containing a nucleic acid to be detected using the MDR1 probe, and for the detection of a polymorphism in a gene-encoding base sequence through Tm value analysis based on the differential melting curve, or instructions that describes various reagents that are contained, or may additionally be contained, in the reagent kit.

### EXAMPLES

The present invention will now be described in detail by way of examples. However, the present invention is not limited to these examples in any way.

### [Example 1]

Using a fully-automated SNP analyzer (trade name: I-DENSY (trademark); manufactured by ARKRAY, Inc.) and a test reagent having the formulation shown in Table 3 below, Tm analysis was performed to verify the performance of the MDR1(1236) probe of a fluorescently labeled oligonucleotide (SEQ ID NO:2). Here, the artificial oligo sequences indicated in SEQ ID NOs:18 and 19 were used as templates.

**[Table 3]**

| (Amount of reaction solution: 50 µl) | |
|---|---|
| 1 × Gene Taq Universal buffer (manufactured by NIPPON GENE CO., LTD.) | |
| MDR1(1236) probe | 0.2 µM |
| Template | 0.2 µM |

Details of the probe and templates used in the above Table 3 are shown below. Here, the type of fluorescent dye is indicated in parentheses at the 3'-end of the probe. In the base sequence shown in Table 4, the capital letter indicates the position of the polymorphism.
The same applies hereinafter.

**[Table 4]**

| | | |
|---|---|---|
| MDR1(1236) probe | ttcaggttcagAcccttc-(TAMRA) | SEQ ID NO:2 |

**[Table 5]**

| Sequence (5'→3') | Length | SEQ ID NO: |
|---|---|---|
| gatcttgaagggTctgaacctgaag | 25 | 18 |
| gatcttgaagggCctgaacctgaag | 25 | 19 |

The Tm analysis was performed by treating the reaction solution at 95°C for 1 second and then at 40°C for 60 seconds and subsequently measuring the change in fluorescence intensity over time during a period in which the temperature of the solution was increased from 40°C to 75°C at a rate of 1°C/3 seconds. Here, the excitation wavelength and the detection wavelength were set in the range of 520 nm to 555 nm and 585 nm to 700 nm, respectively, to measure the changes in fluorescence intensity originating from the respective fluorescently labeled probes.

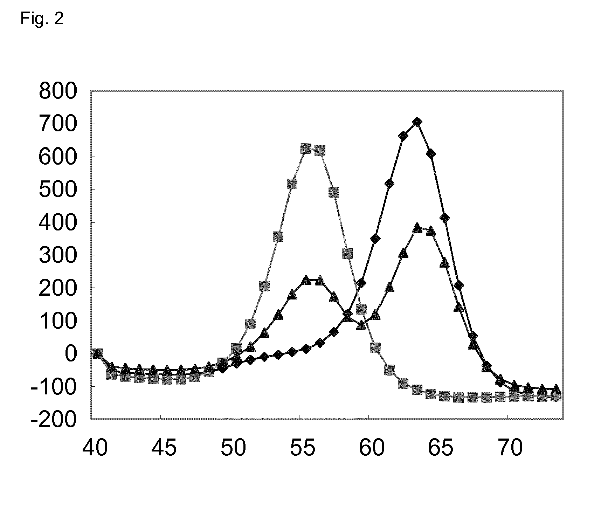

The Tm analysis yielded Fig. 2 showing the changes in the fluorescence value of the respective probes. In Fig. 2, the ordinate indicates the change in fluorescence intensity per unit time (increase in the d-fluorescence intensity/t), and the abscissa indicates the temperature (°C). In Fig. 2, the pattern indicated with diamonds represents the results obtained by using, as a template, the artificial oligo sequence indicated in SEQ ID NO:19 having the same base sequence as indicated in SEQ ID NO:1 except that the 40 1 st base (Y) is C. The pattern indicated with squares represents the results obtained by using, as a template, the artificial oligo sequence indicated in SEQ ID NO:18 having the same base sequence as indicated in SEQ ID NO:1 except that the 40 1 st base (Y) is T, and the pattern indicated with triangles represents the results obtained by using, as a template, a 1:1 mixture of the artificial oligo sequences indicated in SEQ ID NOs:18 and 20.

From the results shown in Fig. 2, it was proven that, by using the fluorescently labeled oligonucleotide according to the present invention as a probe, a polymorphism at the 40 1 st base of the base sequence indicated in SEQ ID NO:1 can be detected even when a sequence having the same bases as indicated in SEQ ID NO:1 except that the 40 1 st base (Y) is C coexists with a sequence having the same bases as indicated in SEQ ID NO:1 except that the 401st base (Y) is T. Further, the Tm value was 60°C when the artificial oligo sequence indicated in SEQ ID NO:19 was used as a template and 56°C when the artificial oligo sequence indicated in SEQ ID NO:18 was used as a template.

### [Example 2]

Using a fully-automated SNP analyzer (trade name: I-DENSY (trademark); manufactured by ARKRAY, Inc.) and a reaction solution having the formulation shown in Table 6 or 7 below, PCR and Tm analysis were performed.

**[Table 6]**

| <<en a purified human genome was used as a template>> | |
|---|---|
| (Amount of reaction solution: 50 µl) | |
| 1 × PCR buffer | |
| dNTP | 0.2 mM |
| MgCl2 | 1.5 mM |
| Taq polymerase (manufactured by ARKRAY, Inc.) | 1.88 U/test |
| MDR1 exon26 probe | 0.2 µM |
| MDR1 exon21 probe 2 | 0.2 µM |
| MDR1(1236) probe | 0.2 µM |
| MDR1 exon21-F | 1 µM |
| MDR1 exon21-R | 0.2 µM |
| MDR1 exon26-F | 1 µM |
| MDR1 exon26-R | 0.2 µM |
| MDR1(1236)F | 1 µM |
| MDR1(1236)R | 0.2 µM |
| Purified human genome | 100 copies |

| | |
|---|---|
| Human genome purified from whole blood was used | |

**[Table 7]**

| <<When whole blood was used as template>> | |
|---|---|
| (Amount of reaction solution: 50 µl) | |
| 1 × PCR buffer | |
| dNTP | 0.2 mM |
| MgCl2 | 1.5 mM |
| Taq polymerase (manufactured by ARKRAY, Inc.) | 1.88 U/test |
| MDR1 exon26 probe | 0.2 µM |
| MDR1 exon21 probe 2 | 0.2 µM |
| MDR1(1236) probe | 0.2 µM |
| MDR1 exon21-F | 1 µM |
| MDR1 exon21-R | 0.2 µM |
| MDR1 exon26-F | 1 µM |
| MDR1 exon26-R | 0.2 µM |
| MDR1(1236)F | 1 µM |
| MDR1(1236)R | 0.2 µM |
| Pre-treated whole blood | 4 µl |

The PCR was performed by treating the reaction solution at 95°C for 1 second and then repeating 50 cycles of 95°C for 1 second and 57°C for 30 seconds.
The Tm analysis was performed after the PCR by treating the reaction solution at 95°C for 1 second and then at 40°C for 60 seconds and subsequently measuring the change in fluorescence intensity over time during a period in which the temperature of the solution was increased from 40°C to 75°C at a rate of 1°C/3 seconds.

As a polymorphism detection probe, a fluorescently labeled oligonucleotide having the same sequence as indicated in SEQ ID NO:2 except that the 3'-end is labeled with a fluorescent dye (TAMRA) (MDR1(1236) probe), a fluorescently labeled oligonucleotide having the same sequence as indicated in SEQ ID NO:9 except that the 5'-end is labeled with a fluorescent dye (PACIFIC BLUE) (MDR1 exon 26 probe) and a fluorescently labeled oligonucleotide having the same sequence as indicated in SEQ ID NO:10 except that the 5'-end is labeled with a fluorescent dye (BODIPY FL) (MDR1 exon 21 probe2) were employed. Details of the MDR1 exon 26 probe and the MDR1 exon 21 probe are shown below.

**[Table 8]**

| Probe | Sequence (5'→3') | SEQ ID NO: |
|---|---|---|
| MDR1 exon26 probe | (PACIFIC BLUE)-ctgccctcacAatctcttc-P | 9 |
| MDR1 exon21 probe2 | (BODIPY FL)-cccagAaccttctagttc-P | 10 |

Here, in the fluorescent dye, PACIFIC BLUE, the excitation wavelength is 365 nm to 415 nm, and the detection wavelength is 445 nm to 480 nm. In the fluorescent dye, BODIPY FL, the excitation wavelength is 420 nm to 485 nm, and the detection wavelength is 520 nm to 555 nm. In the fluorescent dye, TAMRA, the excitation wavelength is 520 nm to 555 nm, and the detection wavelength is 585 nm to 700 nm (the same applies hereinafter). Based on these wavelengths, the changes in fluorescence intensity originating from the respective fluorescently labeled probes were measured.

Among those primers that were used, details of the primers other than the MDR1(1236)F indicated in SEQ ID NO:7 and the MDR1(1236)R indicated in SEQ ID NO:8 are shown below.

**[Table 9]**

| Primer | Sequence (5'→3') | SEQ ID NO: |
|---|---|---|
| MDR1 exon21-F | aaatgttgtctggacaagcactg | 11 |
| MDR1 exon21-R | aattaatcaatcatatttagtttgactcac | 12 |
| MDR1 exon26-F | actgcagcattgctgagaac | 13 |
| MDR1 exon26-R | cagagaggctgccacatgctc | 14 |

As the template, whole blood and human genomic DNA purified from whole blood by a conventional method were employed.
The whole blood was prepared in the following manner.
To 70 µl of diluent 1, 10 µl of whole blood was added and mixed well, and 10 µl of the resulting mixture was then added to 70 µl of diluent 2. By heating 17 µl of the thus obtained mixture at 95°C for 10 minutes, 4 µl of pre-treated whole blood was obtained. This was used as the template per one test.

**[Table 10]**

| Diluent 1 | | | Diluent 2 | |
|---|---|---|---|---|
| Tris-HCl (pH8.0) | 10 mM | | Tris-HCl (pH8.0) | 10 mM |
| EDTA (pH8.0) | 0.1 mM | | 500mM EDTA (pH8.0) | 0.1 mM |
| SDS | 0.30% | | | |

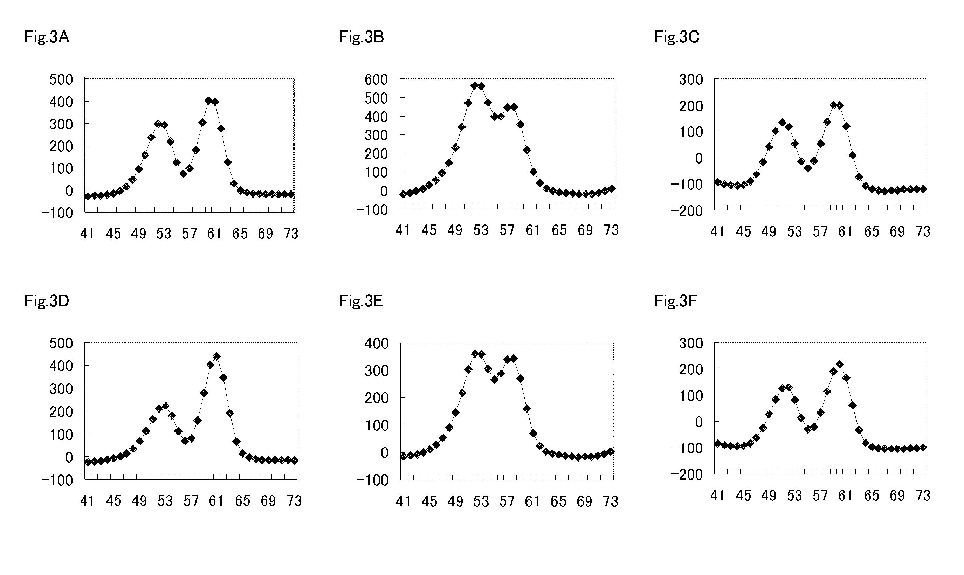

The Tm analysis yielded Fig. 3 showing the changes in the fluorescence value of the respective probes.
Figs. 3(A) to (C) show the results obtained using the purified human genomic DNA as a sample.
Figs. 3(D) to (F) show the results obtained using whole blood as the sample.
Further, Figs. 3(A) and (D) show the presence or absence of the polymorphism corresponding to the 40 1 st base of the base sequence indicated in SEQ ID NO:1, and Figs. 3(B) and (E) show the presence or absence of the polymorphism corresponding to the 256th base of the base sequence indicated in SEQ ID NO:15. Figs. 3(C) and (F) show the presence or absence of the polymorphism corresponding to the 300th base of the base sequence indicated in SEQ ID NO:16.
In these figures, the ordinate indicates the change in fluorescence intensity per unit time (increase in the d-fluorescence intensity/t), and the abscissa indicates the temperature (°C).

As a result, it was proven that, by using the fluorescently labeled oligonucleotide according to the present invention as a probe, a polymorphism at the 40 1 st base of the base sequence indicated in SEQ ID NO:1 can be easily detected simultaneously in the same system with the presence or absence of a polymorphism corresponding to the 256th base of the base sequence indicated in SEQ ID NO:15 and a polymorphism corresponding to the 300th base of the base sequence indicated in SEQ ID NO:16.

### [Comparative Example 1]

Using a fully-automated SNP analyzer (trade name: I-DENSY (trademark); manufactured by ARKRAY, Inc.) and a test reagent having the formulation shown in Table 11 below, Tm analysis was performed. Here, the fluorescently labeled oligonucleotide indicated in SEQ ID NO:17 was used as a probe, and the artificial oligo sequences indicated in SEQ ID NOs:20 and 21 were used as templates.

**[Table 11]**

| (Amount of reaction solution: 50 µl) | |
|---|---|
| 1 × Gene Taq Universal buffer (manufactured bv NIPPON GENE CO.. LTD.) | |
| Probe | 0.2 µM |
| Template | 0.2 µM |

Details of the probe and templates used in the above Table 11 are shown below. Here, the type of fluorescent dye is indicated in parentheses at the 3'-end of the probe.

**[Table 12]**

| Sequence (5'→3') | Length (mer) | Tm value | | A value between C and T | SEQ ID NO: |
|---|---|---|---|---|---|
| | | T | C | | |
| tgaagggTctgaacc-(TAMRA) | 15 | 45 | 39 | 6 | 17 |

**[Table 13]**

| Sequence (5'→3') | Length | SEQ ID NO: |
|---|---|---|
| cttcaggttcagAcccttcaagatc | 25 | 20 |
| cttcaggttcagGcccttcaagatc | 25 | 21 |

The Tm analysis was performed by treating the reaction solution at 95°C for 1 second and then at 40°C for 60 seconds and subsequently measuring the change in fluorescence intensity over time during a period in which the temperature of the solution was increased from 40°C to 75°C at a rate of 1°C/3 seconds. As the fluorescent dye, TAMRA was employed.

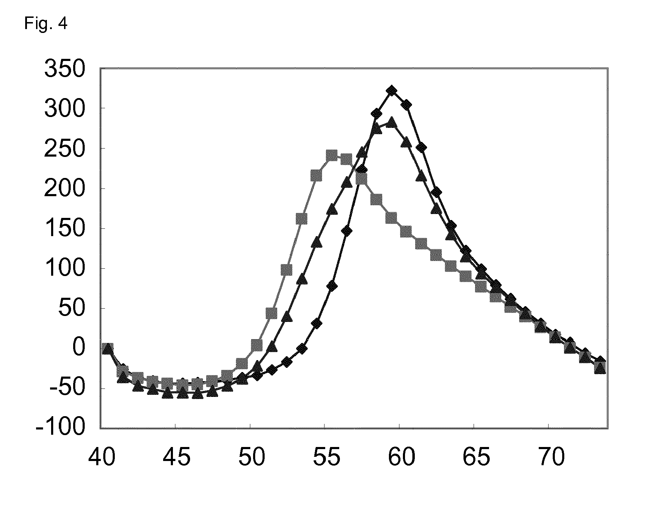

The Tm analysis yielded Fig. 4 showing the changes in the fluorescence value of the respective probes. In Fig. 4, the ordinate indicates the change in fluorescence intensity per unit time (increase in the d-fluorescence intensity/t), and the abscissa indicates the temperature (°C). In Fig. 4, the pattern indicated with diamonds represents the results obtained using, as a template, the artificial oligo sequence indicated in SEQ ID NO:21 having the same base sequence as indicated in SEQ ID NO:1 except that the 40 1 st base (Y) is C. The pattern indicated with squares represents the results obtained using, as a template, the artificial oligo sequence indicated in SEQ ID NO:20 having the same base sequence as indicated in SEQ ID NO:1 except that the 40 1 st base (Y) is T, and the pattern indicated with triangles represents the results obtained using, as a template, a 1:1 mixture of the artificial oligo sequences indicated in SEQ ID NOs:20 and 21.

According to the results shown in Fig. 4, when the sequence having the same bases as indicated in SEQ ID NO:1 except that the 40 1 st base (Y) is C coexisted with the sequence having the same bases as indicated in SEQ ID NO:1 except that the 40 1 st base (Y) is T, only one detection peak was obtained, and a polymorphism could not be detected.
It is noted here that the fluorescently labeled oligonucleotide indicated in SEQ ID NO:17 which was used in Comparative Example 1 was presented merely as a representative example of undetectable probes that exist in large numbers.

From the above-described results, it was demonstrated that, by the present invention, a polymorphism in the MDR1 gene can be detected easily with high sensitivity.

## Claims

1. A probe for detecting a polymorphism in the MDR1 gene, which is a fluorescently labeled oligonucleotide selected from the group consisting of the following P1 and P1':
(P1) an oligonucleotide having an identity of at least 80% to a sequence complementary to a base sequence of 7 to 38 bases in length including the 395th to the 401st bases of the base sequence indicated in SEQ ID NO:1, wherein the base corresponding to said 395th base is a cytosine labeled with a fluorescent dye; and
(P1') an oligonucleotide which hybridizes under stringent conditions to the complementary strand of a sequence that is itself complementary to a base sequence of 7 to 38 bases in length including the 395th to the 401st bases of the base sequence indicated in SEQ ID NO:1, wherein the base corresponding to said 395th base is a cytosine labeled with a fluorescent dye.

2. The probe according to claim 1, which is at least one fluorescently labeled oligonucleotide selected from the group consisting of the following P1-1 and P1'-1:
(P1-1) an oligonucleotide having an identity of at least 80% to a sequence complementary to a base sequence of 7 to 38 bases in length including the 395th to the 401st bases of the base sequence indicated in SEQ ID NO:1, wherein the base corresponding to said 395th base is a cytosine labeled with a fluorescent dye, said oligonucleotide recognizing a polymorphism at the 40 1 st base of SEQ ID NO:1; and
(P1'-1) an oligonucleotide which hybridizes under stringent conditions to the complementary strand of a sequence that is itself complementary to a base sequence of 7 to 38 bases in length including the 395th to the 401st bases of the base sequence indicated in SEQ ID NO:1, wherein the base corresponding to said 395th base is a cytosine labeled with a fluorescent dye, said oligonucleotide recognizing a polymorphism at the 401 st base of SEQ ID NO:1.

3. The probe according to claim 1 or 2, wherein said P1 fluorescently labeled oligonucleotide or P1' fluorescently labeled oligonucleotide comprises the base corresponding to said 395th base labeled with a fluorescent dye at any one of the first to the third positions from the 3'-end, wherein preferably said base is at the 3'-end.

4. The probe according to any one of claims 1 to 3, wherein said P1 fluorescently labeled oligonucleotide or P1' fluorescently labeled oligonucleotide emits fluorescence when it is not hybridized to a target sequence, and the fluorescence intensity of said P1 fluorescently labeled oligonucleotide or P1' fluorescently labeled oligonucleotide when hybridized to said target sequence is decreased or increased as compared to when not hybridized to said target sequence.

5. The probe according to claim 4, wherein said P1 fluorescently labeled oligonucleotide or P1' fluorescently labeled oligonucleotide emits fluorescence when it is not hybridized to a target sequence, and the fluorescence intensity of said P1 fluorescently labeled oligonucleotide or P1' fluorescently labeled oligonucleotide when hybridized to said target sequence is decreased as compared to when not hybridized to said target sequence.

6. The probe according to any one of claims 1 to 5, wherein said P1 fluorescently labeled oligonucleotide or P1' fluorescently labeled oligonucleotide has a length of 7 to 28 bases, preferably 7 to 18 bases.

7. The probe according to any one of claims 1 to 6, which is a probe for melting curve analysis.

8. A method of detecting a polymorphism in the MDR1 gene, the method comprising the steps of:
(I) bringing the probe according to any one of claims 1 to 7 into contact with a single-stranded nucleic acid contained in a sample to allow hybridization of said fluorescently labeled oligonucleotide to said single-stranded nucleic acid, thereby obtaining a hybrid;
(II) dissociating said hybrid by changing the temperature of said sample containing said hybrid to measure the change in fluorescence signal caused by dissociation of said hybrid;
(III) determining the Tm value, which is the dissociation temperature of said hybrid, based on said change in fluorescence signal; and
(IV) based on said Tm value, detecting the presence of a polymorphism of the MDR1 gene in said single-stranded nucleic acid in said sample.

9. The method according to claim 8, which further comprises the step of amplifying said nucleic acid prior to or simultaneously with said step (I).

10. The method according to claim 8 or 9, which further comprises the step of detecting, in the same system, at least one of a polymorphism corresponding to the 256th base of the base sequence indicated in SEQ ID NO:15 or a polymorphism corresponding to the 300th base of the base sequence indicated in SEQ ID NO:16.

11. The method according to any one of claims 8 to 10, which comprises the step of detecting, in the same system, a polymorphism corresponding to the 401 st base of the base sequence indicated in SEQ ID NO:1, a polymorphism corresponding to the 256th base of the base sequence indicated in SEQ ID NO:15 and a polymorphism corresponding to the 300th base of the base sequence indicated in SEQ ID NO:16.

12. A method of evaluating efficacy of a drug, which comprises the steps of:
detecting a polymorphism in the MDR1 gene by the method of detecting a polymorphism according to any one of claims 8 to 11; and
determining the tolerance to said drug or the efficacy of said drug based on the presence or absence of a detected polymorphism.

13. The method according to claim 12 wherein said drug is selected from an anticancer drug, an antihypertensive drug, an immunosuppressive agent and a centrally acting drug.

14. A reagent kit for detecting a polymorphism in the MDR1 gene, which comprises the probe according to any one of claims 1 to 7, preferably wherein said reagent kit further comprises a primer for amplifying a base sequence containing a region to which said probe hybridizes, and/or a probe for detecting a polymorphism corresponding to the 256th base of the base sequence indicated in SEQ ID NO:1 and a probe for detecting a polymorphism corresponding to the 300th base of the base sequence indicated in SEQ ID NO:16.

15. Use of the reagent kit according to claim 14 in the detection of a polymorphism in the MDR1 gene.
